(19) Europäisches Patentamt

European Patent Office

Office européen des brevets

(11) EP 0 841 077 A2

(12) EUROPÄISCHE PATENTANMELDUNG

(43) Veröffentlichungstag:
13.05.1998 Patentblatt 1998/20

(51) Int. Cl.⁶: A61N 5/06

(21) Anmeldenummer: 97117508.8

(22) Anmeldetag: 09.10.1997

(84) Benannte Vertragsstaaten:
AT BE CH DE DK ES FI FR GB GR IE IT LI LU MC
NL PT SE

(30) Priorität: 14.10.1996 DE 19642337

(71) Anmelder: Haslauer, Paul
A-5020 Salzburg (AT)

(72) Erfinder: Hentschel, Hans-Jürgen
D-83301 Traunreut (DE)

(74) Vertreter:
Flach, Dieter Rolf Paul, Dipl.-Phys. et al
Patentanwälte
Andrae Flach Haug Kneissl
Bauer Schneider,
Prinzregentenstrasse 24
83022 Rosenheim (DE)

(54) **Mit Reflektoren versehene Bestrahlungseinrichtung**

(57)     Eine verbesserte Bestrahlungsvorrichtung, insbesondere zur Durchführung einer Licht- oder UV-Bestrahlung, umfaßt eine Reflektor-Anordnung (2'). Mit dieser Bestrahlungsvorrichtung ist es möglich, das Licht auf den Lichtbadenden oder Patienten abzubilden oder zu fokussieren. Dazu weist die Bestrahlungsvorrichtung folgende weitere Merkmale auf

- die zumindest eine Lichtquelle (5, 6, 15, 16) liegt außerhalb des Raumes zwischen der Sekundär-Reflektor-Anordnung (2') und der Abbildungs- bzw. Nutzfläche (1),
- die zumindest eine Lichtquelle (5, 6, 15, 16) und die Sekundär-Reflektor-Anordnung (2') ist derart angeordnet und ausgebildet, daß das von der zumindest einen Lichtquelle (5, 6, 15, 16) ausgestrahlte Licht auf eine Abbildungs- oder Nutzfläche (1) strahlt,
- die Sekundär-Reflektor-Anordnung (2') ist derart, daß die Gesamtabmessung in Quer- und Längsrichtung zumindest 3m x 3m beträgt, und
- der Abstand zwischen der Sekundär-Reflektor-Anordnung (2') und der Abbildungs- und Nutzfläche (1) beträgt zumindest 1,5m.

Fig. 1

EP 0 841 077 A2

**Beschreibung**

Die Erfindung betrifft eine Bestrahlungseinrichtung zur Durchführung einer Licht- oder UV-Bestrahlung nach dem Oberbegriff des Anspruchs 1.

Eine gattungsbildende Bestrahlungsvorrichtung ist beispielsweise aus der US 4,911,166 bekannt geworden. Sie umfaßt zwei Beleuchtungsvorrichtungen, die nebeneinanderliegend an einem Kopfhelm befestigt sind. In den kleinen Beleuchtungskästen ist eine Lampe untergebracht, die von einem rückwärtigen Spiegel und einer frontseitigen Linse in das jeweilige Auge abgebildet wird. Derartige Vorrichtungen dienen zur Lichtbehandlung.

Eine Vorrichtung für eine Bestrahlungsliege ist grundsätzlich auch aus der DE 26 21 476 A1 bekannt geworden. Es handelt sich hier um eine Vorrichtung zur Ganzkörperbestrahlung, bei welcher zum Zwecke einer gleichmäßigen Rundumbestrahlung des Körpers ebene oder rinnenförmig gekrümmte, längliche Reflektorteile seitlich an der Liege vorgesehen sind. Diese Reflektorflächen sind so ausgerichtet, daß sie die von der Strahlungsfläche austretende Strahlung über die seitlichen Reflektoren auf die gegenüberliegenden Seiten eines Patienten reflektiert. Im wesentlichen aber wird der Patient auf der Liege von der Bestrahlungsquelle direkt bestrahlt.

Ein kosmetisches Bestrahlungsgerät ist schließlich auch aus der DE-PS 869 671 bekannt geworden, welches als Ganzkörper-Bestrahlungsgerät dient und ein Grundaufbau vergleichbar mit der eingangs genannte US-Patentschrift 4,911,166 aufweist. Es handelt sich hierbei ebenfalls wieder um ein typisches konstruktiv kompakt gestaltetes einheitlich handhabbares Gerät.

Schließlich sind aus der DE-PS 614 729 auch Reflektoren als Sonnenlicht-Sammler zur Bestrahlung des Gesichtes eines Patienten bekannt. Gemäß der DE 26 21 476 sind Hilfsreflektoren an einer Patientenliege befestigbar. Schließlich ist in der DE 44 11 791 A1 eine Bestrahlungseinrichtung zur Fokussierung von Sonnenlicht auf dem Patienten zu entnehmen, wobei es sich hierbei ebenfalls nur um ein medizinisches Bestrahlungsgerät handelt.

Demgegenüber ist es Aufgabe der vorliegenden Erfindung, ausgehend von dem eingangs genannten Stand der Technik, eine verbesserte Bestrahlungsvorrichtung zur Licht- und UV-Bestrahlung zu schaffen.

Die Aufgabe wird erfindungsgemäß entsprechend den im Anspruch 1 angegebenen Merkmalen gelöst. Vorteilhaft Ausgestaltungen der Erfindung sind in den Unteransprüchen angegeben.

Demgegenüber wird mit der vorliegenden Erfindung ein völlig neuartiger Weg beschritten.

Dabei wird zunächst einmal auf die deutsche Hauptanmeldung P 196 06 396.5 Bezug genommen, die der nachveröffentlichten DE 196 06 396 A1 entspricht. Der Offenbarungsgehalt der vorstehend genannten Anmeldung bzw. nachveröffentlichten Offenlegungsschrift wird in vollem Umfang zum Inhalt der vorliegenden Patentanmeldung gemacht.

Kern der vorliegenden Erfindung ist, daß eine Bestrahlungseinrichtung geschaffen wird, die nicht wie ein herkömmliches kompaktes Bestrahlungsgerät arbeitet, sondern die insgesamt so aufgebaut ist, daß zum einen die Lichtquellen dem direkten Einblick der eine entsprechende Lichtbehandlung durchführenden Person entzogen sind und/oder der Patient sich zudem möglichst frei in einer eigenen "Erlebniswelt" bewegen kann.

Der Eindruck einer direkten Bestrahlung, wie er bei den nach dem Stand der Technik bekannten Bestrahlungsgeräten bekannt ist, wird erfindungsgemäß vermieden.

Dabei sind gemäß der vorliegenden Erfindung bevorzugt die Abstände zwischen den Lichtquellen, d.h. der zumindest einen Lichtquelle beispielsweise in Form von Lampen mit und ohne Hilfsreflektoren, Scheinwerfern etc. zu der Sekundär-Spiegelreflektor-Anordnung und zwischen dieser und dem Patienten frei wählbar, um die Personen, die die Lichtbestrahlung auf sich anwenden wollen, weitgehende Bewegungsfreiheit zu verschaffen und den erwähnten Eindruck einer "Erlebniswelt" zu gewährleisten und aufrechtzuerhalten.

Die erfindungsgemäße Bestrahlungsvorrichtung mit der erwähnten Sekundär-Spiegelreflektor-Anordnung hat dabei gegenüber bekannten Einrichtungen zur Allgemeinbeleuchtung eine das Licht sammelnde, auf einer vergleichsweise groß bemessenen Abbildungs- und Nutzfläche fokussierende Form. Somit kann das Licht in Form einer Ganzkörperbestrahlung auf den gesamten Patienten abgebildet werden.

In Erweiterung des Erfindungsgedankens nach der nicht vorveröffentlichten deutschen Basispatentanmeldung P 196 06 396 wird die Sekundär-Spiegelreflektor-Anordnung so ausgestaltet, daß sie das Licht der zumindest einen Lichtquelle auf eine größer dimensionierte Nutzfläche abbildet, die vorzugsweise für zumindest zwei oder mehrere Patienten ausreicht, das Licht also auf diese Fläche weitgehend konzentriert wird.

Wenn bevorzugt die Abbildungs- und Nutzfläche als horizontale Boden- oder Liegefläche ausgebildet und die Sekundär-Spiegelreflektor-Anordnung nach Art einer Deckenkonstruktion gestaltet ist, erscheint dem Lichtbadenden die Sekundär-Spiegelreflektor-Anordnung quasi als künstlicher Himmel.

Um in einem entsprechend großen Maßstab eine gewünschte Lichtdurchflutung und -abbildung auf der Abbildungs- und Nutzfläche zu gewährleisten, beträgt die Summe der Sektoren oder Segmente der Sekundär-Spiegelreflektor-Anordnung Abmessungen von mindestens 3m x 3m, vorzugsweise 3,5m x 3,5m bis vorzugsweise um 4m x 4m. Die vorstehend genannten Maßangaben entsprechen also den minimalen Maximumsangaben, also beispielsweise einer kreisförmigen Sekundär-Spiegelreflektor-Anordnung mit einem Durchmesser von zumindest 3m, um eine Abmessung in Längs- und Querrichtung von 3m x 3m zu gewährleisten.

Die Lichtquellen sind mit frei wählbaren Abstand außerhalb und unterhalb der Nutzfläche oder in ihr liegend derart angeordnet, daß sie auf die korrespondierende, ggf. mehrgliedrige Sekundär-Spiegelreflektor-Anordnung (welche mehrere Reflektor-Segmente umfaßen kann) gerichtet, vorzugsweise nach oben strahlen und dem direkten Einblick des sich frei bewegenden oder liegenden Patienten entzogen sind.

Bevorzugt können benachbarte Lichtquellen entweder mit Tageslichtlampen oder UV-Strahlern oder einer Kombination von beiden ausgestattet sein, um bei Umschaltung zwischen ihnen wechselnde Bestrahlungen mit Tageslichtintensität von mindestens 1000 Lux oder für eine allmähliche Bräunung ausreichende UV-Bestrahlungsstärken zu ermöglichen.

Zusammenfassend kann also festgehalten werden, daß die vorliegende Erfindung folgende Merkmale und Vorteile umfaßt bzw. in einer oder mehreren bevorzugten Ausgestaltungen der Erfindung nachstehend ebenfalls wiedergegebene Vorzüge erzielbar sind:

- eine Sekundär-Spiegelreflektor-Anordnung kann grundsätzlich einen Basisaufbau aufweisen, wie er aus der nicht vorveröffentlichten P 196 06 396 (der dem Inhalt der nicht vorveröffentlichten DE 196 06 396 A1 entspricht) aufweisen, auf die in vollem Umfang Bezug genommen und zum Inhalt der vorliegenden Anmeldung gemacht wird;

- es sind Lichtquellen und ein Sekundär-Spiegelreflektor vorzugsweise mit mehreren Sektoren oder Segmenten vorgesehen;

- der zumindest eine oder die mehreren den Sekundär-Spiegelreflektoren bildenden Sektoren oder Segmente sind in zumindest einem bis mehreren Querschnitten parabolisch, näherungsweise sphärisch gekrümmt;

- die Sekundär-Spiegelreflektoren bzw. deren Sektoren oder Segmente bilden gemäß der Beziehung

$$1/f = 1/a + 1/b$$

die Lichtquellen gleichmäßig (oder zumindest annähernd gleichmäßig, also zumindest nicht zu stark unterschiedlich hell) auf die Nutz- oder Abbildungsfläche mit dem Patienten oder Lichtbadenden ab, wobei a der Abstand zwischen der zumindest einen zugehörigen Lichtquelle und dem Sekundär-Spiegelreflektor und b der Abstand der Abbildungs- oder Nutzfläche zum Sekundär-Spiegelreflektor entspricht;

- die Sekundär-Spiegelreflektor-Anordnung kann rotationssymmetrisch zu einer vertikalen Achse ausgebildet sein;

- die Sekundär-Spiegelreflektoren können aus mehreren Sektoren zusammengesetzt sein, die ihrerseits durch Schablonen formschlüssig gehalten und vorzugsweise durch ein aus Metallprofilen gebildetes Tragelement zu einer transportablen Einheit zusammengefügt sind;

- die Sekundär-Spiegelreflektoren und damit das sie tragende Tragelement können Abmessungen aufweisen, deren Längs- oder Querabmessung zumindest 3m, vorzugsweise um 4m im Durchmesser entspricht;

- die Sekundär-Spiegelreflektoren können insgesamt eine langgestreckte Form oder einen langestreckten Grundriß aufweisen und dabei beispielsweise zwei halbrunde Teile und zwei rinnenförmig ausgebildete Teile dazwischen aufweisen, die im Querschnitt vorzugsweise eine parabolische, näherungsweise eine sphärische Krümmung mit einer Brennweite f zur Abbildung der zumindest einen Lichtquelle auf die Abbildungs- und Nutzfläche aufweisen;

- im Falle der Verwendung von rinnenförmigen Sekundär-Spiegelreflektoren können diese mit in rinnenförmigen Hilfsreflektoren liegenden Leuchtstofflampen (also mit einer linienförmigen Lichtquelle) oder mit punktartigen Strahlern zusammenwirken;

- insbesondere bei punktförmigen Strahlern können Sekundär-Spiegelreflektoren auch aus einzelnen Segmenten ausgebildet sein, die ihrerseits in sich parabolisch, näherungsweise sphärisch konkav mit einer geeigneten Brennweite f gekrümmt sind, um die Lichtquellen wieder möglichst gleichmäßig über die Abbildungs- und Nutzfläche hinweg verteilt abzubilden.

Die Erfindung wird nachfolgend für Ausführungsbeispiele anhand von Zeichnungen näher erläutert. Dabei zeigen im einzelnen

Figur 1 :  ein erstes Ausführungsbeispiel in schematischer vereinfachter räumlicher Darstellung;

Figur 2 :  eine schematische Vertikalquerschnittsdarstellung durch die erfindungsgemäße Bestrahlungsvorrichtung;

Figur 3 :  eine entsprechende Draufsicht auf die Darstellung gemäß Figur 2;

Figur 4 :  ein abgewandeltes Ausführungsbeispiel in schematischer Draufsicht bzw. in Horizontalquerschnittsdarstellung; und

Figur 5 : ein weiteres Ausführungsbeispiel in schematischer, räumlicher Darstellung.

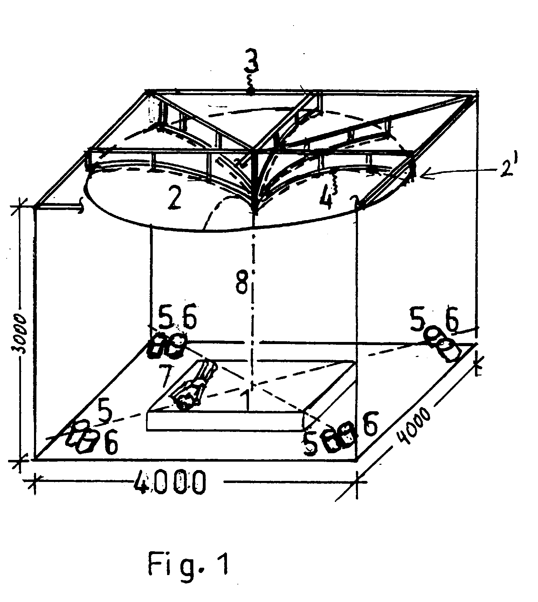

Nachfolgend wird auf Figuren 1 bis 3 Bezug genommen.

In Figur 1 kann ein Lichtbadender, der nachfolgend der Einfachheit halber kurz als Patient 7 bezeichnet wird, sich auf einer Abbildungs- oder Nutzfläche 1 befinden. Die Abbildungs- oder Nutzfläche 1 kann eine quadratische Grundfläche mit einer Seitenlänge von beispielsweise 4m aufweisen. Die Höhe der gesamten Bestrahlungsvorrichtung und -anordnung beträgt beispielsweise 3m.

Eine Sekundär-Spiegelreflektor-Anordnung 2' umfaßt bei diesem Ausführungsbeispiel einen Sekundär-Spiegelreflektor 2, der sich in einer Höhe zwischen 2,5m und 2,8m befindet. Der im Ausführungsbeispiel rotationssymmetrisch zu einer senkrechten Raumachse 8 ausgebildete Reflektor 2 besteht bevorzugt aus einzelnen Sektoren aus einseitig glänzendem und eloxierten Aluminiumblech. Diese Sektoren liegen formschlüssig an Schablonen 4 an, welche mit einem Tragelement 3 aus Metallprofilen zu einer selbständigen transportablen und montierbaren bzw. demontierbaren Einheit verbunden sind, die in Figur 1 nur teilweise dargestellt sind.

In den diagonal gegenüberliegenden Ecken der Anordnung befinden sich Lichtquellen 5, 6, die in diesem Ausführungsbeispiel mit einer Tageslichtlampe als Lichtquelle 5 und einem UV-Strahler als Lichtquelle 6 ausgestattet sind. Die Lichtquellen werfen das Licht gebündelt in an sich bekannter Weise wie Scheinwerferlicht auf den zugeordneten Teil der Sekundär-Reflektoren.

Als UV-Strahler können die Lichtquellen mit geeigneten Filtern versehen sein, um beispielsweise UV-A oder UV-A plus UV-B Lichtanteile durchzulassen. Die Anordnung der Lichtquellen 5, 6 sind außerhalb des Aufenthaltsbereiches 1 des Patienten 7 und in bzw. unterhalb der Ebene der Abbildungs- oder Nutzfläche 1 angeordnet, wodurch verhindert wird, daß ein Patient 7 direkt die Lichtquellen 5, 6 erblicken kann.

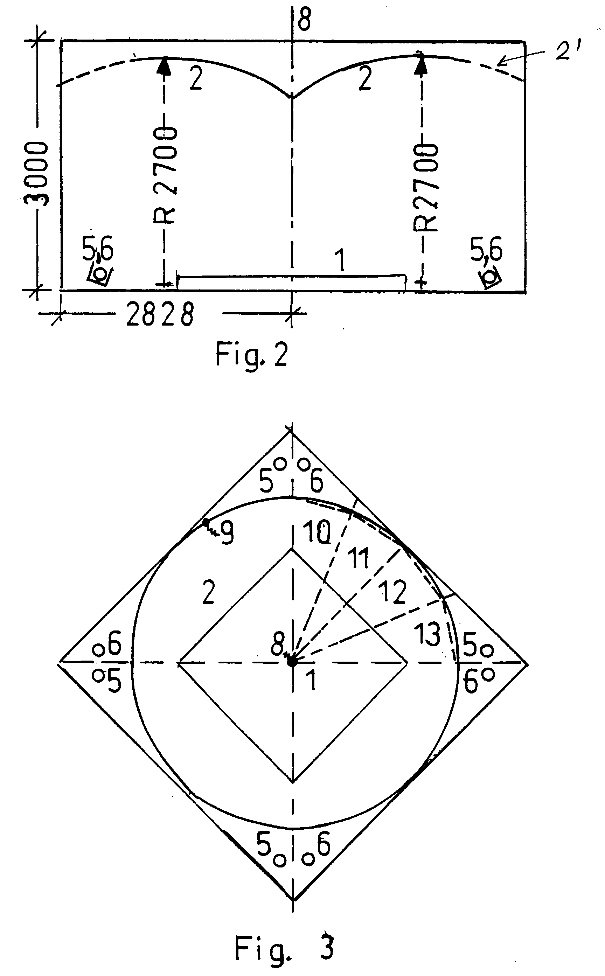

Nachfolgend wird auf Figur 2 Bezug genommen, die einen Vertikalschnitt durch die Anordnung in einer vertikalen Ebene in Figur 1, die längs einer Diagonale durch Figur 1 gelegt ist, wiedergibt. Im dargestellten Schnitt wird der parabolische Querschnitt des Reflektors 2 angenähert durch einen Kreisbogen mit einem Radius R = 2,7m entsprechend einer Brennweite f = 1,35m zur Abbildung der Lichtquellen 5, 6 auf die Abbildungs- und Nutzfläche 1. Dabei wird sowohl durch die nur angenähert parabolische Form des Reflektors 2 als auch durch seine vorzugsweise leichtstreuende Oberfläche die Lichtquelle 5, 6 unscharf abgebildet. Der Reflektor 2 ist im Beispiel rotationssymmetrisch um die Achse 8 der Anordnung ausgebildet, so daß in Ebenen, die nicht die Lichtquellen 5, 6 enthalten, die Abbildung vom angenommenen Ort auf die Abbildungs- oder Nutzfläche 1 weg verlagert wird. Dadurch und durch die Überlagerung der Abbildung aller Lichtquellen 5, 6 oder weiterer Lichtquellen wird die Abbildungs- oder Nutzfläche 1 und ihre Umgebung gleichmäßig bestrahlt, d.h. in dem gewünschten Maß zumindest ausreichend gleichmäßig bestrahlt.

Nachfolgend wird Bezug genommen auf Figur 3, in welcher eine Grundrißdarstellung bezüglich des Ausführungsbeispiels nach Figur 2 wiedergegeben ist. Aus Figur 3 ist auch die Lage der Lichtquellen 5, 6, die Lage des zur Achse 8 rotationssymmetrischen Reflektors mit seinem Umfang 9 sowie der Grundriß der Abbildungs- und Nutzfläche 1 ersichtlich. Bevorzugt ist die Reflektor-Anordnung 2 aus Sektoren 10, 11, 12 und 13 - im gezeigten Ausführungsbeispiel für einen Quadranten des kreisförmigen Reflektors dargestellt - die entweder als gleichgroße Sektoren bis zum Umfang 9 zugeschnitten, oder wie aus Figur 2 ersichtlich, bis in die diagonalen Ecken heruntergezogen sein können. Wie in Figur 1 dargestellt, sind die Sektoren 10, 11, 12 und 13 in ihrer Form durch Schablonen, d.h. entsprechend geformte Tragelemente, stabilisiert und befestigt.

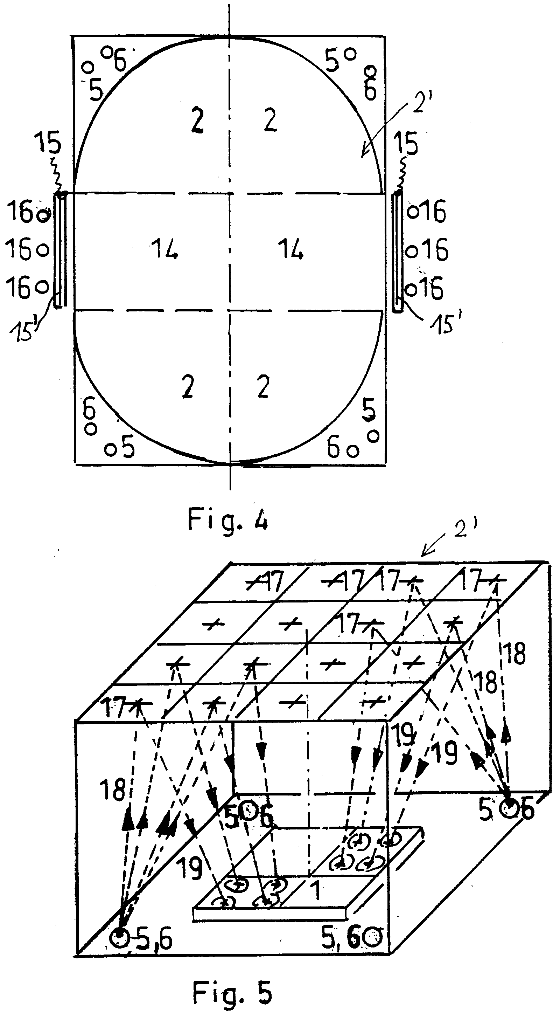

In Figur 4 ist ein weiteres Ausführungsbeispiel einer Sekundär-Spiegelreflektor-Anordnung mit einem Sekundär-Spiegelreflektor über einem rechteckigen Grundriß wiedergegeben.

Die beiden Enden der Abbildungs- und Nutzfläche 1 werden beispielsweise über halbrunde Teile des Reflektors 2 wie nach den Figuren 1 bis 3 bestrahlt. Der mittlere, dazwischen eingefügte Teil weist rinnenförmige Sekundär-Spiegelreflektoren 14 mit einer sphärischen Krümmung quer zur Längsachse mit dem Radius R nach Figur 2 auf. Bevorzugt können hier auch Leuchtstofflampen in an sich bekannten rinnenförmigen Hilfsreflektoren 15 liegend neben Strahlern 16 verwendet werden.

Figur 5 zeigt ein weiteres abgewandeltes Ausführungsbeispiel, bei welchem der Sekundär-Spiegelreflektor 2 in einzelne Segmente 17 aufgeteilt ist. Jedes der Reflektor-Segmente 17 ist an sich vorzugsweise sphärisch konkav mit einer derartigen Brennweite geformt, daß die Lichtquellen 5, 6 auf der Abbildungs- und Nutzfläche 1 abgebildet werden. Dabei lenkt jedes Segment 17 das Lichtbündel 18 von den Lichtquellen 5, 6 in Richtung 19 auf einen Teil der Nutzfläche 1 um, die damit - zumindest weitgehend - gleichmäßig ausgeleuchtet erscheint. Die Abmessungen des Ausführungsbeispiels können dabei denen gemäß Figur 1 entsprechen.

Mit der erläuterten Bestrahlungsvorrichtung wird zudem der Leistungsaufwand gegenüber einer an sich bekannten Allgemeinbeleuchtung eines Raumes mit diffus reflektierender Decke mit Tageslichtintensitäten vergleichbar erheblich verringert. Auch die Möglichkeit einer UV-Bestrahlung ist mit einer Allgemeinbeleuchtung nicht möglich. Ferner stellt die beanspruchte Sekundär-Spiegelreflektor-Anordnung eine Bestrahlungseinrichtung dar, die unabhängig vom Raum transportabel montiert und genutzt werden kann.

**Patentansprüche**

1. Bestrahlungsvorrichtung, insbesondere zur Durchführung einer Licht- oder UV-Bestrahlung mit den folgenden Merkmalen

   - mit einer Lichtquellen-Anordnung mit zumindest einer Lichtquelle (5, 6, 15, 16), welche vorzugsweise mit jeweils einem Hilfsreflektor versehen ist,
   - mit einer Sekundär-Reflektor-Anordnung (2') mit zumindest einem Sekundär-Spiegelreflektor zur Reflektion des von der zumindest einen Lichtquelle (5, 6, 15, 16) kommenden Lichtes,
   - die Abbildungs- oder Nutzfläche (1) liegt in dem Raum vor der zumindest einen Reflektor (2) umfassenden Sekundär-Reflektor-Anordnung (2'),
     **gekennzeichnet durch** die folgenden weiteren Merkmale
   - die zumindest eine Lichtquelle (5, 6, 15, 16) liegt außerhalb des Raumes zwischen der Sekundär-Reflektor-Anordnung (2') und der Abbildungs- bzw. Nutzfläche (1),
   - die zumindest eine Lichtquelle (5, 6, 15, 16) und die Sekundär-Reflektor-Anordnung (2') ist derart angeordnet und ausgebildet, daß das von der zumindest einen Lichtquelle (5, 6, 15, 16) ausgestrahlte Licht auf eine Abbildungs- oder Nutzfläche (1) strahlt,
   - die Sekundär-Reflektor-Anordnung (2') ist derart, daß die Gesamtabmessung in Quer- und Längsrichtung zumindest 3m x 3m beträgt, und
   - der Abstand zwischen der Sekundär-Reflektor-Anordnung (2') und der Abbildungs- und Nutzfläche (1) oder einer vom Licht bestrahlten Person beträgt zumindest 1,5m.

2. Bestrahlungsvorrichtung nach Anspruch 1, **dadurch gekennzeichnet,** daß die Abbildungs- und Nutzfläche (1) nach Art einer horizontalen Bodenfläche gestaltet ist, und die Sekundär-Reflektor-Anordnung (2') als künstlicher Himmel nach Art einer Deckenkonstruktion gebildet ist.

3. Bestrahlungsvorrichtung nach Anspruch 1 oder 2, **dadurch gekennzeichnet,** daß der Abstand zwischen der zumindest einen Lichtquelle (5, 6, 15, 16) und der zugehörigen Sekundär-Reflektor-Anordnung (2') mindestens einen Meter, vorzugsweise um drei Meter beträgt.

4. Bestrahlungsvorrichtung nach einem der Ansprüche 1 bis 3, **dadurch gekennzeichnet,** daß der Abstand zwischen der Sekundär-Reflektor-Anordnung (2') und der Abbildungs- oder Nutzfläche (1) vorzugsweise mehr als 2,5 m beträgt.

5. Bestrahlungsvorrichtung nach einem der Ansprüche 1 bis 4, **dadurch gekennzeichnet,** daß der Abstand zwischen der Sekundär-Reflektor-Anordnung (2') und der Abbildungs- oder Nutzfläche (1) vorzugsweise 2,5 - 3 m beträgt.

6. Bestrahlungsvorrichtung nach einem der Ansprüche 1 bis 5, **dadurch gekennzeichnet,** daß die Sekundär-Reflektor-Anordnung (2') rotationssymmetrisch zu einer Vertikalachse (8) gebildet ist.

7. Bestrahlungsvorrichtung nach einem der Ansprüche 1 bis 6, **dadurch gekennzeichnet,** daß die Sekundär-Reflektor-Anordnung (2') aus mehreren Sektoren (10, 11, 12, 13) zusammengesetzt ist.

8. Bestrahlungsvorrichtung nach einem der Ansprüche 1 bis 7, **dadurch gekennzeichnet,** daß die Sekundär-Reflektor-Anordnung (2') zwei halbrunde Reflektor-Teile (2) umfaßt, zwischen denen zwei rinnenförmige Teilreflektoren (14) sind.

9. Bestrahlungsvorrichtung nach Anspruch 8, **dadurch gekennzeichnet,** daß die rinnenförmigen Sekundär-Reflektoren (14) einen parabolischen, näherungsweise sphärisch gekrümmten Querschnitt aufweisen.

10. Bestrahlungsvorrichtung nach einem der Ansprüche 7 bis 9, **dadurch gekennzeichnet,** daß die rinnenförmigen Sekundär-Reflektoren (14) mit einer Lichtquellen-Anordnung zusammenwirken, die aus zumindest einer Leuchtstofflampe (15') oder längs einer Linie punktartig hintereinandersitzenden Strahlern (16) bestehen.

11. Bestrahlungsvorrichtung nach einem der Ansprüche 1 bis 10, **dadurch gekennzeichnet,** daß die Sekundär-Reflektor-Anordnung (2') aus einer Vielzahl von Reflektor-Segmenten (17) besteht, die jeweils in sich parabolisch, näherungsweise sphärisch konkav gekrümmt sind.

12. Bestrahlungsvorrichtung nach einem der Ansprüche 1 bis 11, **dadurch gekennzeichnet,** daß die einzelnen Sekundär-Reflektoren (2, 17) mit einer Brennweite f gekrümmt sind, wobei die Brennweite f in Bezug auf die Lichtquellen-Anordnung derart gewählt ist, daß das von der Lichtquellen-Anordnung ausgestrahlte Licht zumindest ansatzweise gleichmäßig über die Abbildungs- und Nutzfläche (1) verteilt abgebildet wird.

**13.** Bestrahlungsvorrichtung nach einem der Ansprüche 1 bis 12, **dadurch gekennzeichnet,** daß die gesamte Bestrahlungsvorrichtung einschließlich der Sekundär-Spiegelreflektor-Anordnung (2') transportabel ausgestaltet ist.

**14.** Bestrahlungsvorrichtung nach einem der Ansprüche 1 bis 13, **dadurch gekennzeichnet,** daß die Lichtquellen (5, 6, 15, 16) außerhalb der Abbildungs- und Nutzfläche (1) angeordnet sind.

**15.** Bestrahlungsvorrichtung nach einem der Ansprüche 1 bis 14, **dadurch gekennzeichnet,** daß die Lichtquellen (5, 6, 15, 16) in oder unterhalb der Ebene der Abbildungs- oder Nutzfläche (1) angeordnet sind.

**16.** Bestrahlungsvorrichtung nach einem der Ansprüche 1 bis 15, **dadurch gekennzeichnet,** daß die Lichtquellen (5, 6, 15, 16) wechselweise mit Tageslichtlampen und UV-Strahlern ausgerüstet und umschaltbar sind.

**17.** Bestrahlungsvorrichtung nach einem der Ansprüche 1 bis 16, **dadurch gekennzeichnet,** daß der Sekundär-Reflektor (2) Sektoren (10, 11, 12, 13) oder Segmente (17) vorzugsweise aus glänzendem, elektrolytisch oxidiertem Aluminium umfaßt oder daraus besteht und die Spiegeloberfläche leicht streuend mattiert ist.

**18.** Sekundär-Reflektoranordnung, insbesondere Sekundär-Spiegelreflekoranordnung, für eine Bestrahlungsvorrichtung entsprechend einem der vorstehend genannten Ansprüche 1 bis 17.

Fig. 1

Fig. 2

Fig. 3

Fig. 4

Fig. 5